(19) Europäisches Patentamt
European Patent Office
Office européen des brevets

(11) **EP 4 426 265 B1**

(12) **EUROPEAN PATENT SPECIFICATION**

(45) Date of publication and mention
of the grant of the patent:
**22.04.2026 Bulletin 2026/17**

(21) Application number: **22814509.0**

(22) Date of filing: **07.11.2022**

(51) International Patent Classification (IPC):
*A61K 9/00* (2006.01)     *A61K 47/02* (2006.01)
*A61K 47/06* (2006.01)     *A61K 47/10* (2017.01)
*A61K 47/36* (2006.01)     *A61K 47/38* (2006.01)
*A61K 47/32* (2006.01)     *A61K 31/7032* (2006.01)
*A61K 31/728* (2006.01)    *A61K 33/22* (2006.01)
*A61K 36/85* (2006.01)     *A61P 1/00* (2006.01)

(52) Cooperative Patent Classification (CPC):
(C-Sets available)
**A61K 9/0048; A61K 31/7032; A61K 31/728;**
**A61K 33/22; A61K 36/85; A61K 47/02;**
**A61K 47/10; A61K 47/36; A61P 1/00;** A61K 47/06;
A61K 47/32; A61K 47/38           (Cont.)

(86) International application number:
**PCT/IB2022/060682**

(87) International publication number:
**WO 2023/079520 (11.05.2023 Gazette 2023/19)**

(54) **OPHTHALMIC COMPOSITION COMPRISING A LUBRICANT AGENT AND VERBASCOSIDE, AND SAID COMPOSITION FOR USE IN THE PREVENTIVE OR CURATIVE TREATMENT OF A CORNEAL EPITHELIAL DEFECT AND/OR A CONJUNCTIVAL DEFECT IN A SUBJECT**

OPHTHALMISCHE ZUSAMMENSETZUNG MIT GLEITMITTEL UND VERBASCOSIDE, UND DIESE IN DER VERWENDUNG ZUR PRÄVENTION ODER HEILUNG EINES KORNEALEN EPITHELIALDEFEKTS UND/ODER EINES KONJUNKTIVALEN DEFEKTS BEI EINEM PATIENTEN

COMPOSITION OPHTALMIQUE COMPRENANT UN AGENT LUBRIFIANT ET DU VERBASCOSIDE, ET CETTE COMPOSITION POUR SON UTILISATION DANS LE TRAITEMENT PRÉVENTIF OU CURATIF D'UNE LÉSION ÉPITHÉLIALE DE LA CORNÉE ET/OU D'UNE LÉSION CONJONCTIVALE CHEZ UN SUJET

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.11.2021 IT 202100028220**

(43) Date of publication of application:
**11.09.2024 Bulletin 2024/37**

(73) Proprietor: **NOVAX PHARMA S.A.M.**
**98000 Monaco (MC)**

(72) Inventor: **DIAS FERREIRA, Victor**
**98000 Monaco (MC)**

(74) Representative: **Benedetto, Marco**
**Marben S.r.l.**
**Via Larga, 16**
**20122 Milano (IT)**

(56) References cited:
**WO-A1-2012/158591     IT-A1- MI20 091 165**

(52) Cooperative Patent Classification (CPC): (Cont.)

C-Sets
**A61K 31/7032, A61K 2300/00;**
**A61K 31/728, A61K 2300/00;**
**A61K 33/22, A61K 2300/00;**
**A61K 36/85, A61K 36/00**

**Description**

The present invention relates to an ophthalmic composition comprising a mixture that comprises or, alternatively, consists of: (a) at least one lubricant agent wherein said (a) lubricant agent is a mixture of (a.1) a hyaluronic acid or a salt thereof and (a.10) a polyethylene glycol (PEG) with an average molecular weight comprised from 200 Da to 600 Da; (b) verbascoside, isoverbascoside or mixtures thereof; and, optionally, (c) at least one pharmaceutically acceptable additive.

Further, the present invention relates to said ophthalmic composition for use in a method for the preventive or curative treatment of a disorder or a disease associated with a corneal epithelial defect and/or a conjunctival defect in a subject.

Eye drops are often the only possible administration route for treating and curing certain diseases, for example in case of superficial abrasions and irritation.

However, an effective amount of the drug administered through a drop of an eye drop is unfortunately very uncertain. In particular, there are unintentional defence mechanisms of the eye - such as lachrymation and blinking - that constantly expel foreign bodies (such as dust and fibres) from the eye, so that most of the administered drop goes lost from the intended target eye area. The lost amount can be up to 85% of a dispensed amount in a very short time.

In addition to that, an eye drop has a volume comprised from 30 $\mu$l to 35 $\mu$l so that multiple daily administrations are often necessary, and more drops (2-3) are necessary for each daily administration. Further, the number of daily administrations has to be increased to compensate the tear washout and the small volume of each drop.

All these circumstances can lead to an increased risk that a subject will not adhere to the therapeutic treatment.

Document WO2012/158591 A1 is directed to a cosmetic composition and method for treating or preventing blepharitis. Said composition comprises verbascoside and shale oil. The shale oil can be present in a concentration ranging from 0.0001% by weight to about 10% by weight of the total composition. The shale oil is useful as an anti-inflammatory, antibacterial and antimycotic effect.

Document ITMI20091165 A1 discloses an ophthalmic composition comprising an extract of *Lippia citriodora* and hyaluronic acid, optionally with PEG and preservatives.

The Applicant, after a long and intensive research and development program, has developed a composition capable of providing an adequate response to the existing limitations, drawbacks and problems which are still present in the state of the art. In particular, the Applicant has found a composition capable of mixing with tears and forming a viscous and transparent performed layer that, by reducing the friction caused by eye movements and blinking, protects the surface of the eye therefore promoting wound and abrasion healing.

In the context of the present invention, the compositions according to the present invention do not contain shale oil, Sodium Sulfonated Shale Oil, such as any shale oil suitable for topical use including light-colored pale sulfonated shale oil (ICHTHYOL® pale) and dark sulfonated shale oil (ICHTHYOL®).

In the contest of the present invention, the compositions according to the present invention do not contain high molecular weight polyethylene glycol (PEG) such as PEG-9000 or PEG-14000.

A first object of the present invention is provided by an ophthalmic composition comprising a mixture that comprises or, alternatively, consists of: (a) at least one lubricant agent wherein said (a) lubricant agent is a mixture of (a.1) a hyaluronic acid or a salt thereof and (a.10) a polyethylene glycol (PEG) with an average molecular weight comprised from 200 Da to 600 Da; (b) verbascoside, isoverbascoside or mixtures thereof; and, optionally, (c) at least one pharmaceutically acceptable additive, having the features as per the appended claims.

In the context of the present invention, the term "composition" means for example a pharmaceutical composition or a medical device composition according to the European regulation on medical devices [UE 2017/745 -(MDR), Directive 93/42/CEE -(MDD)].

Another object of the present invention relates to said ophthalmic composition for use in a method for the preventive or curative treatment of a disorder or a disease associated with a corneal epithelial defect and/or a conjunctival defect in a subject, having the features as per the appended claims.

Preferred embodiments of the present invention will be described below by way of a non-limiting example, with the aid of the accompanying drawing, showing:

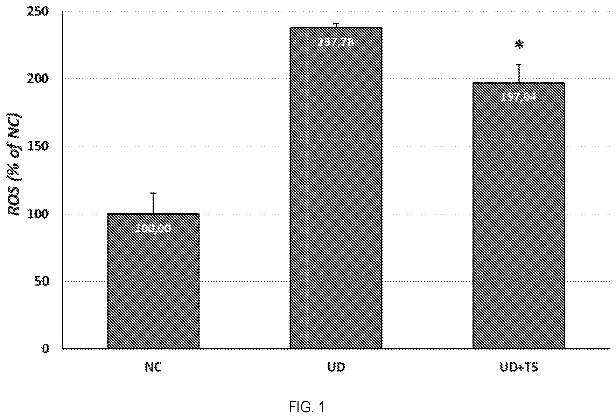

-    Figure 1: a diagram relating to the absorption test of verbascoside and boric acid discussed in Example 2, wherein the values are expressed as means $\pm$ standard deviation of three (3) determinations. Percentages were calculated with respect to the florescence values of untreated and unstimulated tissues. Statistical data processing was performed by Student's t-test. We considered significative values of $p < 0.05$. NC = negative control, untreated and not stimulated cells; UD = cells stimulated with urban dust; UD + TS = cells treated with the tested sample and stimulated with urban dust. * $p < 0.05$ vs UD.

As said above, an ophthalmic composition represents an object of the present invention.

The ophthalmic composition is preferably a liquid ophthalmic composition, more preferably is a liquid sterile ophthalmic composition.

More preferably, said ophthalmic composition is in the form of liquid eye drops, or gel eye drops, cream eye drops or ointment eye drops, preferably in the form of liquid eye drops.

Even more preferably, said liquid composition is an aqueous liquid composition. An aqueous liquid composition comprises water as a solvent, preferably purified water or water for injection (WFI).

Said aqueous liquid composition preferably has a pH value (measured at room temperature of 25°C and at pressure of 1 atm) comprised from 6.8 to 7.4, preferably of 7.2 ± 1.

Said aqueous liquid composition is preferably an isotonic composition, with an osmolality comprised from 250 mOsm/kg to 350 mOsm/kg.

More preferably, said aqueous liquid composition has a density (measured at a room temperature of 25°C) comprised from 1,000 g/ml to 1,020 g/ml, and a viscosity - measured on the freshly prepared composition - comprised from 19,00 cSt and 39,00 cSt.

The measurement of said density can be performed by means of a pycnometer of known volume and an analytical balance according to the following procedure: P1 and P2 are respectively the weight of the empty pycnometer of known volume (V) and the weight of said pycnometer filled with the product to be tested. P1 and P2 are measured using a calibrated analytical balance. The density (D) of the product is calculated as:

$$D = (P2 - P1) / V$$

The measurement of said viscosity can be performed with a capillary viscometer according to the following procedure and parameters: (1) Viscosity measurement is carried out at a temperature of 20°C ± 1°C; (2) A capillary viscometer according to the reference viscosity range is chosen; (3) Cleaning and absence of water in the three arms of a viscometer (viscosimeter "Ubbelohde" according to said appropriate reference viscosity range (delivered by VWR International GmbH - Darmstadt (Germany)) are verified; (4) The viscometer with the product to be tested is rinsed; (5) The viscometer is filled with the product solution to be tested so that the level in the reservoir is between the lines of maximum and minimum level; (6) The viscometer is immersed in a thermostatic system brought to the chosen temperature (20°C ± 1°C) till a thermal equilibrium has been reached; (7) A conical end of the viscometer is inserted into the three ways pro-pipette (i.e. aspirate the solution) and the solution is drawn up until the start measurement time zero mark has been passed by at least 1 cm, while keeping the other arm closed; (8) The time taken by the liquid to cover the stretch of viscometer defined by the two marks is recorded; (9) the viscosity is calculated with the equation:

$$\eta = k \times t$$

wherein $\eta$ is the dynamic viscosity expressed in centiStoke (cSt), k is the constant declared by the viscometer manufacturer, and t is the recorded time expressed in seconds.

Said composition is preferably substantially free of phosphate ions, i.e. free of phosphate ions, monohydrogen phosphate ions and/or dihydrogen phosphate ions.

In the present description the expression "substantially free of phosphate ions" means a total absence of phosphate ions, monohydrogen phosphate ions and/or dihydrogen phosphate ions in the composition and/or mixture, or a presence of phosphates at an amount comprised from greater than zero to 7 mmol/litre, preferably from greater than zero to 3 mmol/litre, even more preferably from greater than zero to 1 mmol/litre of phosphate ions.

Said ophthalmic composition comprises a mixture that comprises or, alternatively, consists of:

   (a) at least one lubricant agent selected from the group that comprises or, alternatively, consists of the following lubricant agents from (a.1) to (a.14): (a.1) a hyaluronic acid or a salt thereof; (a.2) hydroxypropylmethyl cellulose; (a.3) carboxymethyl cellulose or a salt thereof; (a.4) hydroxyethyl cellulose; (a.5) methyl cellulose; (a.6) polyvinyl alcohol (PVA) or a salt thereof; (a.7) polyvinylpyrrolidone (PVP); (a.8) polyacrylic acid or a salt thereof; (a.9) petrolatum optionally in combination with at least one selected from the group comprising or, alternatively, consisting of: lanolin, anhydrous lanolin, mineral oil, light mineral oil, paraffin, white wax, yellow wax, and mixtures thereof; (a.10) a polyethylene glycol (PEG) with an average molecular weight comprised from 200 Da to 600 Da; (a.11) propylene glycol; (a.12) glycerine; (a.13) polysorbate-80; and (a.14) any mixture of (a.1)-(a.13); The claimed invention comprises a lubricant agent which is a mixture of (a.1) and (a.10).
   (b) verbascoside, isoverbascoside or mixtures thereof; preferably verbascoside; and optionally
   (c) at least one pharmaceutically acceptable additive.

In the composition of the present invention, the lubricant agent (a) increases the viscosity, and it also improves the mucoadhesivity properties of the composition. In this way, (b) verbascoside, isoverbascoside or mixtures thereof has/have prolonged residence time and effects on the eye surface.

When the composition of the present invention is applied to the conjunctival fornix, said composition is mixed with tears. After mixing it forms a viscous, transparent and subtle layer that reduces the friction caused by the eye movements and blinking. Working in this way, it protects the surface of the eye therefore promoting wound and abrasion healing.

An amount of said (a) lubricant agent in the composition of the present invention is preferably comprised from 0.05% to 5% by weight, more preferably comprised from 0.1% to 3% by weight, even more preferably comprised from 0.15% to 1.5% by weight, with respect to the overall weight of said composition. Hyaluronic acid (hereinafter referred to as HA) products are used in eye surgery. The viscoelastic properties of hyaluronic acid are used to support structures in the eye and prevent them from collapsing during surgery. Hyaluronic acid HA is not only biocompatible but an important natural constituent of healthy eyes. Moreover, HA can be modified in different ways to improve its properties such as molecular weight, viscosity, and hydrophobicity to adapt the new artificial molecule to different aims.

In the composition of the present invention, hyaluronic acid or salt thereof (a.1), preferably having an average molecular weight comprised from 5.000 kDa to 10.000 kDa, preferably comprised from 6.000 kDa to 8.000 kDa, more preferably comprised from 6.500 kDa to 7.500 kDa, even more preferably comprised from 6.800 kDa to 7.200 kDa, is in combination with (a.10), (b), and optionally (c) Preferably, hyaluronic acid or salt thereof (a.1) is obtained by fermentation of bacteria of the genus *Streptococcus*, more preferably of the species *Streptococcus zooepidermicus.*

In a preferred embodiment, said salt of hyaluronic acid (a.1) is sodium hyaluronate.

Said (a.1) hyaluronic acid or salt thereof is preferably a crosslinked hyaluronic acid or salt.

Usually, eye drops containing hyaluronic acid loose viscosity as a function of time and generally arrive to the patient with decreased action and effectiveness. Hyaluronic acid is also heat-sensitive, whereby heat reduces the viscosity and retention time of hyaluronic acid on the corneal surface.

In spite of this, the crosslinked hyaluronic acid (a.1) used in the composition of the present invention is heat stable, and resistant to heat-induced degradation.

Also, crosslinked hyaluronic acid seems to provide better ocular comfort than "linear" (not crosslinked) hyaluronic acid in ophthalmic compositions.

Said (a.1) hyaluronic acid or salt thereof is preferably crosslinked with 1-ethyl-3-(3-dimethylaminopropyl) carbodiimide hydrochloride (EDC).

A crosslinking grade of said (a.1) hyaluronic acid or salt - expressed as a ratio [equivalents of EDC]:[equivalents of free COOH groups of (a.1)] - is preferably comprised from 0.5% to 1.2%, more preferably comprised from 0.65% to 1.0%, even more preferably comprised from 0.75% to 0.85%. Hydroxypropylmethyl cellulose (or hypromellose) is a semisynthetic, inert, viscoelastic polymer used as eye drops, as well as an excipient and controlled-delivery component in oral medicaments, found in a variety of commercial products.

When applied to an ocular surface, a hypromellose solution acts to swell and absorb water, thereby expanding the thickness of the tear film. Hypromellose augmentation therefore results in extended time on the cornea, which results in a decreased eye irritation.

A non-claimed composition comprises (a.2) hydroxypropylmethyl cellulose in combination with (b), and optionally (c).

Said (a.2) hydroxypropylmethyl cellulose preferably comprises an amount of metoxyl groups comprised from 28% to 30% by weight, and/or an amount of hydroxypropoxyl groups comprised from 7,0% to 12,0% by weight.

Said (a.2) hydroxypropylmethyl cellulose is preferably in powder form comprising powder particles, wherein said powder particles preferably have a mean particle size distribution comprised from 0,01 $\mu$m to 500 $\mu$m, preferably comprised from 0,05 to 400 $\mu$m.

Preferably, said (a.2) hydroxypropylmethyl cellulose is the substance with CAS N. 9004-64-03.

A non-claimed composition comprises (a.3) carboxymethyl cellulose or a salt thereof in combination with (b), and optionally (c).

Carboxymethyl cellulose (CMC, or cellulose gum) is a cellulose derivative with carboxymethyl groups (-CH2-COOH) bound to some of the hydroxyl groups of the glucopyranose monomers that make up the cellulose backbone. The number of hydrogen atoms in hydroxyl group of glucose unit replaced by carboxymethyl groups can be expressed by a degree of substitution (DS). If all the three hydroxyl groups in each glucose unit are replaced by carboxymethyl, the DS is equal to 3. The degree of substitution has a direct impact on the solubility, emulsibility, thickening property, stability, acid resistance and salt tolerance.

In the carboxymethyl cellulose or a salt thereof (a.3) used in the composition of the present invention, the average degree of substitution (DS) is preferably comprised from 0.2 to 1.5, more preferably from 0.4 to 1.2, even more preferably comprised from 0.6 to 0.95.

Preferably, the composition of the present invention comprises a sodium salt of carboxymethyl cellulose ("carmellose") in combination with (b) and optionally (c). More preferably, the sodium salt of carboxymethyl cellulose has a sodium content comprised from 1.0% to 15% by weight, preferably comprised from 5.0% to 10% by weight, even more preferably comprised from 6.5% to 9.0% by weight.

**EP 4 426 265 B1**

Even more preferably, a sodium salt of carboxymethyl cellulose is one of the substances CAS N. 74811-65-7 or CAS N. 9004-32-4.

Said carboxymethyl cellulose or salt thereof (a.3) is preferably crosslinked with 1-ethyl-3-(3-dimethylaminopropyl) carbodiimide hydrochloride (EDC) or with 1,4-butanediol diglycidyl ether (BDDE).

A crosslinking grade of said (a.3) carboxymethyl cellulose or salt thereof - expressed as a ratio [equivalents of EDC]:[equivalents of free COOH groups of (a.3)] or as a ratio [equivalents of BDDE]:[equivalents of free COOH groups of (a.3)] - is preferably comprised from 0.5% to 1.2%, more preferably comprised from 0.65% to 1.0%, even more preferably comprised from 0.75% to 0.85%.

A non-claimed composition comprises (a.4) hydroxyethyl cellulose in combination with (b), and optionally (c).

Hydroxyethyl cellulose is a gelling and thickening agent derived from cellulose. It is widely used in cosmetics, cleaning solutions, and other household products.

In the composition of the present invention (a.4) hydroxyethyl cellulose preferably is the substance CAS N. 9004-62-0.

Preferably, said hydroxyethyl cellulose (a.4) has a moisture content comprised from 0.01% to 5.0%, more preferably comprised from 0.02% to 4.0%.

Said hydroxyethyl cellulose (a.4) is preferably a medium viscosity hydroxyethyl cellulose. In particular, a Brooksfield viscosity of a 2% solution of said hydroxyethyl cellulose (a.4) is preferably comprised from 4,500.00 mPa.s to 6,500.00 mPa.s (whereby said viscosity is tested as specified in the Ph. Eur.).

A non-claimed composition comprises (a.5) methyl cellulose in combination with (b), and optionally (c).

Methyl cellulose (or methylcellulose) is another chemical compound derived from cellulose. It is sold under a variety of trade names and is used as a thickener and emulsifier in various food and cosmetic products. Methyl cellulose is not digestible, not toxic, and not an allergen.

In the composition of the present invention (a.5) methyl cellulose (also named methyl ether of cellulose, or partly O-methylated cellulose, or E 461) preferably is a synthetic methyl cellulose obtained from vegetable raw materials, preferably from wood cellulose.

Preferably, said (a.5) methyl cellulose has a weight loss on drying comprised from 0.01% to 5,0%, said weight loss on drying being determined according to the Ph. Eur.

Preferably, said (a.5) methyl cellulose is the substance CAS N. 9004-67-5.

A non-claimed composition comprises (a.6) polyvinyl alcohol (PVA, or PVAC) in combination with (b), and optionally (c).

PVA is a water-soluble synthetic polymer used in papermaking, textile warp sizing, as a thickener and emulsion stabilizer in PVAC adhesive formulations, in a variety of coatings, and in 3D printing technology.

In the composition of the present invention, (a.6) polyvinyl alcohol (PVA) has a degree of hydrolysis comprised from 85.00% to 92.00%, preferably comprised from 87.00% to 89.00%, said degree of hydrolysis being determined according to standard methods.

A non-claimed composition comprises (a.7) polyvinylpyrrolidone (PVP) in combination with (b), and optionally (c).

Polyvinylpyrrolidone (also named polyvidone or povidone) is a water-soluble polymer made from the monomer N-vinylpyrrolidone. PVP is used as a plasma volume expander for trauma victims, as a binder in many pharmaceutical tablets, and in some contact lenses and their packaging solutions. It reduces friction, thus acting as a lubricant, or wetting agent, built into the lens.

In the composition of the present invention, (a.7) polyvinylpyrrolidone (chemical name: alfa-Hydro-omega-hydropoly[1-(2-oxopyrrolidin-1-yl)ethylene] preferably is the substance CAS N. 9003-39-8).

A non-claimed composition comprises (a.8) polyacrylic acid or a salt thereof in combination with (b), and optionally (c).

Polyacrylic acid (PAA) is a derivative of acrylic acid, used as a superabsorbent for detergents and dispersants. Other applications involve paints and cosmetics. PAA stabilizes suspended solids in liquids, prevents emulsions from separating, and controls the consistency in flow of cosmetics.

In the composition of the present invention, (a.8) polyacrylic acid or a salt thereof preferably is sodium polyacrylate (chemical name: 2-propenoic acid sodium salt; preferably 2-propenoic acid, homopolymer, sodium salt; CAS N. 9003-04-7).

A non-claimed composition comprises, in combination with (b), and optionally (c), (a.9) petrolatum optionally in combination with at least one selected from the group comprising or, alternatively, consisting of: lanolin (e.g. CAS N. 8006-54-0), anhydrous lanolin, mineral oil (e.g. CAS N. 8012-95-1 or CAS N. 8042-47-5), light mineral oil, paraffin (e.g. CAS N. 8002-74-2), white wax or white beeswax (e.g. CAS N. 8012-89-3), yellow wax or yellow beeswax (e.g. CAS N. 8012-89-3).

Petrolatum (or vaseline, white vaseline, petroleum jelly, white petrolatum, soft paraffin, or multi-hydrocarbon) is a semi-solid mixture of hydrocarbons with a number of carbon atoms equal to or higher than 25, originally promoted as a topical ointment for its healing properties, currently used for cosmetic purposes.

In the composition of the present invention, (a.9) petrolatum has a congealing point (determined according to ASTM D 938) comprised from 50.0°C to 56.0°C, and a drop point (determined according to Ph. Eur. 2.2.17/B) comprised from 35.0°C to 70.0°C.

More preferably, said (a.9) petrolatum has a cone penetration at 25°C (according to ASTM D 937) comprised from 140 1/10 mm to 160 1/10 mm, and a kinematic viscosity at 100°C (according to ASTM D 7042) comprised from 5.0 mm2/s to 9.0 mm2/s.

Said lanolin, that is in combination with petrolatum, (b) and optionally (c) in said composition, preferably is anhydrous lanolin. More preferably, said lanolin has a saponification value comprised from 90.0 mgKOH/g to 105.0 mgKOH/g and/or a melting point comprised from 38.0°C to 44.0°C. Even more preferably, said lanolin has a paraffin content ≤ 1.0%.

A drop point of said lanolin is preferably comprised from 38.0°C to 44.0°C, and a iodine number comprised from 18.00% to 36.00%.

Said mineral oil (Paraffinum liquidum), that is in combination with petrolatum, (b) and optionally (c) in said composition, preferably is a clear, odourless liquid with a density (at 15°C) comprised from 0.8600 g/ml to 0.8740 g/ml, with a viscosity (at 40°C) comprised from 62.00 mPa.s to 80.00 mPa.s, and a viscosity (at 100°C) comprised from 8.50 mPa.s to 10.00 mPa.s.

Said white beeswax, that is in combination with petrolatum, (b) and optionally (c) in said composition, preferably has a drop point comprised from 61.0°C to 66.0°C, and/or a saponification index comprised from 87.0 mgKOH/g to 104.0 mgKOH/g, and/or an esters index comprised from 70.0 mgKOH/g to 80.0 mgKOH/g. Even more preferably said white beeswax is the substance CAS N. 8012-89-3.

The composition of the present invention comprises (a.10) a polyethylene glycol (PEG) with an average molecular weight comprised from 200 Da to 600 Da in combination with (a.1), (b), and optionally (c)

Polyethylene glycols (PEGs) are polymers of ethylene oxide with the general formula $H\text{-}(O\text{-}CH_2\text{-}CH_2)_n\text{-}OH$, where n is the average number of oxyethylene groups. PEGs may be named based on the molecular weight of the compound. For example, polyethylene glycol 400 (PEG 400) is a polyethylene glycol compound where 400 is the average molecular weight of the compound.

The polyethylene glycol (PEG) in the composition of the present invention preferably has an average molecular weight comprised from 250 Da to 450 Da, preferably comprised from 280 Da to 430 Da, more preferably of 300 Da (PEG 300) and/or 400 Da (PEG 400).

In the composition of the present invention, PEG(s) enhance(s) synergistically mucoadhesivity and viscosity of said (a.1) hyaluronic acid or salt thereof.

In other words, said (a) lubricant agent is a mixture of (a.1) a hyaluronic acid or a salt thereof and (a.10) a polyethylene glycol (PEG) with an average molecular weight comprised from 200 Da to 600 Da, preferably comprised from 250 Da to 450 Da, more preferably comprised from 280 Da to 430 Da, even more preferably of 300 Da (PEG 300) and/or 400 Da (PEG 400).

PEG 300 contained in the present composition preferably has an average molecular weight comprised from 280 Da to 320 Da.

PEG 400 contained in the present composition preferably has an average molecular weight comprised from 380 Da to 420 Da.

A possible method employed for average molecular weight of PEGs is a calculation of the hydroxyl value (OH val.) that is defined as the number of grams or milligrams of potassium hydroxide required to neutralize the acetic acid taken up on acetylation of one gram of a chemical substance that contains free hydroxyl groups.

A Dalton unit (Da) is approximately equal to the molar mass of the same expressed in grams per mole (1 Da ≈ 1 g/mol).

PEG 400 preferably has a solidification point (assessed according to Ph. Eur.) comprised from 4,0°C to 8,0°C, and a relative density (e.g. determined with a pycnometer of known volume and an analytical balance, according to the procedure described herein before) preferably comprised from 1,18 Kg/l to 1,25 Kg/l, more preferably comprised from 1,2 Kg/l to 1,23 Kg/l, even more preferably of 1,210 Kg/l.

More preferably, PEG 400 is the substance with CAS N. 25322-68-3.

PEGs are considered "adhesion promotors" because they facilitate mucoadhesion via *interpenetration.* Interpenetration of polymer chains is an important mucoadhesion mechanism: when the polymer gets into contact with the mucus, the concentration gradient at the interface provokes the spontaneous diffusion of the polymer chains into the mucus layer, and also the diffusion of the mucin glycoproteins into the polymer. Preferably, a weight ratio (a.1):(a.10) is comprised from 5:1 to 1:5, more preferably comprised from 2:1 to 1:2, even more preferably comprised from 1.5:1 to 1:1.5, further preferably of 1:1.

A non-claimed composition comprises (a.11) propylene glycol in combination with (b), and optionally (c). Reference is made to Handbook of Pharmaceutical Excipients Eighth edition, Edited by Paul J Sheskey et al.

Propylene glycol (propane-1,2-diol) is a viscous, colourless liquid classed as a diol. Propylene glycol is used for food applications and for cosmetics and pharmacology purposes.

In the composition of the present invention, said (a.11) propylene glycol preferably has a density (at 25°C) comprised from 1.0350 g/ml to 1.0380 g/ml. Even more preferably, said (a.11) propylene glycol is the substance 1,2-propanediol (CAS N. 57-55-6).

A non-claimed composition comprises (a.12) glycerine in combination with (b), and optionally (c).

Glycerine (named also glycerol) is a polyol compound that makes the backbone of glycerides. Due to having antimicrobial and antiviral properties, glycerine is FDA approved wound and burn treatments.

In the composition of the present invention, (a.12) glycerine is preferably obtained from vegetable raw materials and, more preferably, has a density (at 20°C) comprised from 1.250 g/ml to 1.263 g/ml. Even more preferably, said (a.12) is glycerine propane-1,2,3-triol (CAS N. 56-81-5).

A non-claimed composition comprises (a.13) polysorbate-80 in combination with (b), and optionally (c).

Polysorbate 80 (IUPAC name: Polyoxyethylene (20) sorbitan monooleate) is a non-ionic surfactant and emulsifier used in foods and cosmetics.

In the composition of the present invention, (a.13) polysorbate-80 preferably has a iodine value comprised from 18.000 g I2/100 g to 24.000 g I2/100 g, and/or a saponification value comprised from 45.000 mgKOH/g to 55.000 mgKOH/g, and/or a hydroxyl value comprised from 65.000 mgKOH/g to 80.000 mgKOH/g (all these parameters being measured according to Ph. Eur.). Even more preferably, said (a.13) polysorbate-80 is the substance polyoxyethylene 20 sorbitan monooleate (CAS N. 9005-65-6).

In addition to said (a) at least one lubricant agent, the composition of the present invention also comprises (b) verbascoside, isoverbascoside or mixtures thereof, preferably verbascoside, in said a mixture. Verbascoside (also known as acteoside) is a phenylpropanoid glycoside that is a water-soluble derivative of natural polyphenols widely distributed in the plant kingdom. Verbascoside is structurally characterized by caffeic acid and 4,5-hydroxyphenylethanol bound to a β-(D)-glucopyranoside, with a rhamnose in sequence (1-3) to the glucose molecule.

In the composition of the present invention, verbascoside and/or isoverbascoside promote(s) skin repair and ameliorates skin inflammation due to its/their ROS scavenging, antioxidant, iron chelating, and glutathione transferase (GST) activity inducing properties.

Preferably, said (b) verbascoside, isoverbascoside or mixtures is an extract of a plant of the species *Lippia citriodora*. More preferably said plant extract is a dry plant extract of the leaves of *Lippia citriodora*.

In the present description, "dry" means that said plant extract has a solvent content comprised from 0,01% to 10% by weight, preferably comprised from 0,05% to 8% by weight, with respect to the overall weight of said extract.

Preferably, said dry plant extract has a weight loss on drying comprised from 0,1% to 6% with respect to the overall weight of said extract.

Preferably, said dry plant extract of the leaves of *Lippia citriodora* has the following components (amounts determined with HPLC with respect to the overall weight of said plant extract):

- verbascoside: comprised from 25.00% to 35.00%, preferably comprised from 25.00% to 30.00%; and
- isoverbascoside: comprised from 1.00% to 10.00%, preferably comprised from 1.00% to 5.00%.

A weight ratio (a):(b) is preferably comprised from 10:1 to 1:5, preferably comprised from 5:1 to 1:2, more preferably comprised from 3:1 to 1:1, even more preferably of 2:1.

Said (c) at least one pharmaceutically acceptable additive, contained in said mixture with (a) and (b), is preferably selected from the group comprising or, alternatively, consisting of: a preservative (preferably boric acid), at least one buffering agent (preferably sodium tetraborate and/or potassium hydroxide), and at least one osmotic pressure regulator (preferably selected from the group comprising or, alternatively, consisting of: potassium chloride, calcium chloride, magnesium chloride, sodium chloride, and mixtures thereof), and mixtures thereof.

Said ophthalmic composition preferably comprises (the following amounts being expressed as amount by weight of each component with respect to the overall weight of said composition, unless otherwise specified):

(a) said hyaluronic acid or salt thereof, preferably sodium hyaluronate: from 0.05% to 5.0%, preferably from 0.1% to 2.0%, more preferably from 0.15% to 0.8%, even more preferably from 0.2% to 0.4%;

(a.10) a polyethylene glycol (PEG) with an average molecular weight comprised from 200 Da to 600 Da, preferably PEG 300 or PEG 400, more preferably PEG 400: from 0.05% to 5.0%, preferably from 0.1% to 2.0%, more preferably from 0.15% to 0.8%, even more preferably from 0.2% to 0.4%;

(b) verbascoside, isoverbascoside or mixtures thereof, preferably verbascoside, more preferably a dry plant extract of the leaves of *Lippia citriodora*: from 0.025% to 3.0%, preferably from 0.05% to 1.5%, more preferably from 0.08% to 0.4%, even more preferably from 0.1% to 0.3%;

(c.1) a preservative, preferably boric acid: from 0.1% to 10.0%, preferably from 0.2% to 4.0%, more preferably from 0.3% to 2.0%, even more preferably from 0.7% to 0.9%;

(c.2) at least one buffering agent, preferably sodium tetraborate and/or potassium hydroxide: up to a pH value comprised from 6.8 to 7.4;

(c.3) at least one osmotic pressure regulator, preferably selected from the group comprising or, alternatively, consisting of: potassium chloride, calcium chloride, magnesium chloride, sodium chloride, and mixtures thereof: up to an osmolality comprised from 250 mOsm/kg to 300 mOsm/kg;

(d) water, preferably purified water: up to 100%.

Preferably, said (c.1) boric acid has a weight loss on drying (over silica gel, 5 h; determined according to Ph. Eur.) comprised from 0.01% to 0.5%.

More preferably, said (c.1) boric acid has a freezing point of 171°C and a density of 1.510 g/cm3, both determined according to Ph. Eur.

Preferably, said (c.2) sodium tetraborate is of mineral or synthetic origin, and is preferably free of raw materials of animal origin. More preferably, said (c.2) sodium tetraborate is sodium tetraborate decahydrate (CAS N. 1303-96-4).

More preferably, said (c.2) sodium tetraborate decahydrate has a freezing point of $\geq 62°C$, and/or a boiling point of 1.575°C, and a density of 1.73 g/ml, all determined according to Ph. Eur.

Preferably, said (c.3) potassium chloride has a weight loss on drying comprised from 0.01% to 1.00%, determined according to Ph. Eur. Even more preferably, said (c.3) potassium chloride is the substance CAS N. 7447-40-7.

Preferably, said (c.3) calcium chloride is anhydrous calcium chloride. Even more preferably, said (c.3) calcium chloride is the substance CAS N. 10035-04-8 or CAS N. 10043-52-4.

Preferably, said (c.3) magnesium chloride is the substance CAS N. 7791-18-6.

Preferably, said (c.3) sodium chloride has a weight loss on drying comprised from 0.01% to 0.50%, determined according to Ph. Eur. Even more preferably, said (c.3) sodium chloride is the substance CAS N. 7647-14-5.

Forming another object of the present invention is said ophthalmic composition for use in a method for the preventive or curative treatment of a disorder or a disease associated with a corneal epithelial defect and/or a conjunctival defect in a subject.

Said disorder or disease is selected from the group comprising or, alternatively, consisting of: dry keratitis, xerophthalmia, dry eye syndrome (keratoconjunctivitis sicca), Sjogren's syndrome and Stevens-Johnson syndrome.

Preferably, said disorder or disease is caused by air pollution, more preferably by urban dust (UD) pollution.

The following examples are provided as a non-limiting illustration of the present invention.

EXAMPLES.

EXAMPLE 1A: Qualitative-quantitative example of the composition according to the present invention

A qualitative and quantitative example of a composition according to the present invention is shown in Table 1 below.

Table 1

| .INGREDIENT | USE | % (wt/wt) |
|---|---|---|
| Purified water (d) | solvent | up to 100 |
| Boric acid (c.1) | preservative | 0.80 |
| Sodium tetraborate (c.2) | buffering agent | 0.10 |
| Crosslinked sodium hyaluronate (a.1) | lubricant agent | 0.30 |
| PEG 400 (a.10) | lubricant agent | 0.30 |
| Verbascoside (b) | plant dry extract | 0.15 |
| Potassium hydroxide (c.2) | buffering agent | up to pH 6.8 - 7.4 |
| Potassium chloride (c.3) | osmotic pressure regulator | 0.062 |
| Calcium chloride (c.3) | osmotic pressure regulator | 0.002 |
| Magnesium chloride (c.3) | osmotic pressure regulator | 0.0025 |
| Sodium chloride (c.3) | osmotic pressure regulator | up to 250 - 300 mOsm/kg |

The composition of the present invention as set out above in Table 1 has the chemical-physical parameters indicated in the following Table 1A.

Table 1A

| Aspect | Clear solution, viscous, amber yellow color with characteristic odor |
|---|---|
| pH | 6.80 - 7.40 |
| Density | 1.000 - 1.020 g/ml |
| Viscosity (20°C) | 19.00 - 39.00 cSt |
| Osmolality | 250 - 350 mOsm/kg |

EXAMPLE 1B: Qualitative-quantitative example of other compositions, which are not according to the present invention

Additional qualitative and quantitative examples of compositions, which are not according to the present invention are giver in Tables from 1B to 1D below.

Table 1B

| INGREDIENT | USE | % (wt/wt) |
|---|---|---|
| Purified water (d) | solvent | up to 100 |
| Boric acid (c.1) | preservative | 0.82 |
| Sodium tetraborate (c.2) | buffering agent | 0.11 |
| Crosslinked sodium salt of carboxymethyl cellulose (a.3) | lubricant agent | 0.02-0.4 |
| Verbascoside (b) | plant dry extract | 0.15 |
| Potassium hydroxide (c.2) | buffering agent | up to pH 6.8 - 7.4 |
| Potassium chloride (c.3) | osmotic pressure regulator | 0.063 |
| Calcium chloride (c.3) | osmotic pressure regulator | 0.003 |
| Magnesium chloride (c.3) | osmotic pressure regulator | 0.0028 |
| Sodium chloride (c.3) | osmotic pressure regulator | up to 250 - 300 mOsm/kg |

Table 1C

| INGREDIENT | USE | % (wt/wt) |
|---|---|---|
| Purified water (d) | solvent | up to 100 |
| Boric acid (c.1) | preservative | 0.78 |
| Sodium tetraborate (c.2) | buffering agent | 0.08 |
| Polyvinyl alcohol (PVA) (a.6) | lubricant agent | 0.02-4.0 |
| Verbascoside (b) | plant dry extract | 0.14 |
| Potassium hydroxide (c.2) | buffering agent | up to pH 6.8 - 7.4 |
| Potassium chloride (c.3) | osmotic pressure regulator | 0.060 |
| Calcium chloride (c.3) | osmotic pressure regulator | 0.004 |
| Magnesium chloride (c.3) | osmotic pressure regulator | 0.0023 |
| Sodium chloride (c.3) | osmotic pressure regulator | up to 250 - 300 mOsm/kg |

Table 1D

| INGREDIENT | USE | % (wt/wt) |
|---|---|---|
| Purified water (d) | solvent | up to 100 |
| Boric acid (c.1) | preservative | 0.79 |
| Sodium tetraborate (c.2) | buffering agent | 0.08 |
| Petrolatum (a.9) | lubricant agent | 0,05-10,0 |
| Mineral oil, or paraffin, or white wax (a.9) | lubricant agent | 0,05-10,0 |
| Verbascoside (b) | plant dry extract | 0.16 |
| Potassium hydroxide (c.2) | buffering agent | up to pH 6.8 - 7.4 |
| Potassium chloride (c.3) | osmotic pressure regulator | 0.061 |
| Calcium chloride (c.3) | osmotic pressure regulator | 0.003 |
| Magnesium chloride (c.3) | osmotic pressure regulator | 0.0026 |
| Sodium chloride (c.3) | osmotic pressure regulator | up to 250 - 300 mOsm/kg |

EXAMPLE 2: Absorption test of verbascoside and boric acid

To study the barrier effect of the tested product, its ability to reduce oxidative stress (ROS production) induced by a standard of urban pollution on reconstructed human ocular epithelium (RHOCL) was evaluated. The tissues were treated with the product to be tested and then stimulated with urban dust (UD), while as reference we considered tissues stimulated with urban dust and not treated. The ROS produced were finally measured.

It was observed that in the tissues treated with the tested product the ROS levels are reduced by 17.13% compared to untreated tissues, and this indicates the presence of a protective action against atmospheric pollution.

The aim of the test was to evaluate the protective action of the tested product on reconstructed human ocular epithelium (RHOCL).

MATERIALS AND METHODS

Tissues

Reconstructed cornea-like tissue (RHOCL) construct is a nonkeratinized epithelium prepared from normal human keratinocytes. It models the cornea epithelium with progressively stratified, but not cornified cells.

Tested substances

INGREDIENTS

Composition of the present invention according to Example 1 (Table 1) (briefly, "VERBALUX composition" or "VERBALUX PF").

ROS ASSAY

To measure the amount of ROS produced we used the 2',7'-dichlorodihydrofluorescein diacetate (DCFDA), a fluorogenic probe which can provide robust and reliable measurements of mitochondrial production of free radicals in cells. The DCFDA was dissolved in dimethyl sulfoxide (DMSO) at the concentration of 50 mM and then diluted in the appropriate buffer at the final concentration of 250 $\mu$M. The tissues were incubated with the DCFDA solution for 30 minutes under standard culture conditions. The DCFDA was then removed, the tissues and then treated with the tested sample and stimulated with urban dust. The urban dust is a standard reference material containing polycyclic aromatic hydrocarbons (PAHs), nitro-substituted PAHs, polychlorinated biphenyl (PCB), chlorinated pesticides and inorganic heavy metals. The standard was prepared from atmospheric particulate material collected in the Washington DC area over a period longer than 12 months using a baghouse specially designed for the purpose. The urban dust standard is not intended to be representative of the area in which it was collected, but it should generally typify atmospheric particulate matter obtained from an urban area.

A series of tissues has not been treated, but only stimulated with urban dust. Control tissues were neither stimulated nor treated. At the end of the treatment period, the tissues were washed in PBS and a fluoride reading was performed at the excitation wavelength of 485 nm and at the emission of 530 nm.

RESULTS

The results obtained are summarised in Table 2 below and in Figure 1.

Table 2

| TREATMENT | ROS (% of NC) |
|---|---|
| NC | $100.0 \pm 15.71$ |
| UD | $237.8 \pm 3.14$ |
| UD + TS | $197.0 \pm 13.58$ |

These values are expressed as means $\pm$ standard deviation of three (3) determinations. Percentages were calculated with respect to the florescence values of untreated and unstimulated tissues. Statistical data processing was performed by Student's t-test. We considered significative values of $p < 0.05$. NC = negative control, untreated and not stimulated cells; UD = cells stimulated with urban dust; UD + TS = cells treated with the tested sample and stimulated with urban dust. * $p < 0.05$ vs UD.

In tissues treated with the tested product, the oxidative stress (% ROS) is significantly lower than the oxidative stress of untreated tissues. This indicates that the tested product has a protective action against urban pollution (protective effect of 17.13%).

CONCLUSIONS

The VERBALUX composition plays a protective effect from urban pollution on reconstructed human ocular epithelium.

In presence of the composition of the present invention the level of ROS induced by a standard of urban pollution is significantly reduced.

EXAMPLE 3: Estimation of systemic absorption of hyaluronic acid and boric acid in view of Ramsay E, Del Amo EM, Toropainen E, Tengvall-Unadike U, Ranta VP, Urtti A, Ruponen M, "Corneal and conjunctival drug permeability: Systematic comparison and pharmacokinetic impact in the eye" (Eur J Pharm Sci. 2018 Jul) Drug bioavailability in the aqueous humour was simulated to be <5% and trans-conjunctival systemic absorption was 34-79% of the dose. Loss of drug across the conjunctiva to the blood circulation restricts significantly ocular drug bioavailability and, therefore, ocular absorption does not increase proportionally with the increasing corneal drug permeability.

If we consider the bioavailability and the systemic absorption of the main active constituents of the composition of the present invention, we can calculate the percentages of following Table 3.

Table 3

| INGREDIENT | % (W/W) | AQUEOUS HUMOR (max 5%) | SISTEMIC TC* ABSORPTION (max 79%) |
|---|---|---|---|
| Crosslinked sodium hyaluronate | 0.30 | 0.015 | 0.011 |
| Boric acid | 0.80 | 0.04 | 0.031 |
| *TC = trans-conjunctival | | | |

The main constituents of the VERBALUX composition are non-toxic and without significant metabolic activity, given the context of use of the product.

Furthermore, the processes of absorption of substances through the ocular tissues are limited by the structure of the organ, considerably reducing the absorption of any substances. In particular, the absorption of hyaluronic acid is further slowed by the large size of the molecule (comprised from 5.000 kDa to 10.000 kDa).

Considering this, only a small amount of substances undergoes absorption phenomena, but this has no effect from a safety point of view as well as from the activity of the composition.

It can therefore be concluded that the VERBALUX composition is to be considered safe for the user, and that it is not necessary to carry out further absorption tests.

CONCLUSIONS:

- the percentage average value (%av) of permeability of VERBASCOSIDE in the specific formulation of the medical device Verbalux PF (batch 23072019, expiry date 07/2021) is not measurable since the values are out of the limit of quantification of the analytical method;
- the %av of permeability of BORIC ACID in the specific formulation of the medical device Verbalux PF (batch 23072019, expiry date 07/2021) is 0.9% compared to the initial nominal quantity of the substance in the composition of the present invention.

Advantageously, the composition of the present invention supports the physiological healing process of cornea wounds and abrasions, in particular in case of traumatic events or surgical procedures. Advantageously, the use of the composition of the present invention is indicated for patients at risk of infection.

Advantageously, verbascoside in the composition of the present invention is able to protect ocular tissue and fluids from naturally occurring or pollution-induced oxidation of the cornea. The topical administration of verbascoside reduces significantly the first stage of the wound healing process and abrasions of the corneal epithelium, *a fortiori* when verbascoside is used in combination with (a) a lubricant agent which is a mixture of (a.1) and (a.10).

Advantageously, the presence of electrolytes ($Cl^-$, $Na^+$, $K^+$, $Ca^{2+}$ and/or $Mg^{2+}$) in the composition of the present invention is useful for the cellular biochemical processes and helps to keep the ocular surface in good physiological conditions.

Advantageously, the composition of the present invention provides an anti-pollution barrier effect because:

- it provides a relief of burning and irritation of the eye due to exposure to pollution factors (such as urban dust) and dryness of the eyes;
- it acts as a protector to prevent irritation and relief of dry eye conditions.

**Claims**

1. An ophthalmic composition comprising a mixture that comprises or, alternatively, consists of:

   (a) at least one lubricant agent, wherein said (a) lubricant agent is a mixture of
   (a.1) a hyaluronic acid or a salt thereof and
   (a.10) a polyethylene glycol (PEG) with an average molecular weight comprised from 200 Da to 600 Da;
   (b) verbascoside, isoverbascoside or mixtures thereof; and optionally
   (c) at least one pharmaceutically acceptable additive.

2. The ophthalmic composition according to claim 1, wherein said (a.1) hyaluronic acid or a salt thereof, preferably has an average molecular weight comprised from 5.000 kDa to 10.000 kDa, preferably comprised from 6.000 kDa to 8.000 kDa, more preferably comprised from 6.500 kDa to 7.500 kDa, even more preferably comprised from 6.800 kDa to 7.200 kDa.

3. The ophthalmic composition according to claim 1 or 2, wherein said (a.1) hyaluronic acid or salt thereof is crosslinked with 1-ethyl-3-(3-dimethylaminopropyl) carbodiimide hydrochloride (EDC).

4. The ophthalmic composition according to any one of claims 1-3, wherein said composition is for use in a method for the preventive or curative treatment of a disorder or a disease associated with a corneal epithelial defect and/or a conjunctival defect in a subject which is selected from the group comprising or, alternatively, consisting of: dry keratitis, xerophthalmia, dry eye syndrome (keratoconjunctivitis sicca), Sjogren's syndrome and Stevens-Johnson syndrome.

5. The ophthalmic composition for use according to claim 4, wherein said disorder or disease is caused by air pollution, preferably by urban dust (UD) pollution.

6. The ophthalmic composition according to any one of claims 1-3, or the ophthalmic composition for use according to claims 4 and 5, wherein a weight ratio (a):(b) is comprised from 10:1 to 1:5, preferably comprised from 5:1 to 1:2, more preferably comprised from 3:1 to 1:1, even more preferably of 2:1.

7. The ophthalmic composition according to claims 1-3, 6 or the ophthalmic composition for use according to claims 4-6, wherein said (a) lubricant agent is a mixture of (a.1) a hyaluronic acid or a salt thereof; and (a.10) a polyethylene glycol (PEG) with an average molecular weight comprised from 200 Da to 600 Da, preferably comprised from 250 Da to 450 Da, more preferably comprised from 280 Da to 430 Da, even more preferably of 300 Da (PEG 300) and/or 400 Da (PEG 400).

8. The ophthalmic composition according to claims 1-3, 6, 7 or the ophthalmic composition for use according to claims 4-7, wherein a weight ratio (a.1):(a.10) is preferably comprised from 5:1 to 1:5, preferably comprised from 2:1 to 1:2, more preferably comprised from 1.5:1 to 1:1.5, even more preferably of 1:1.

9. The ophthalmic composition according to claims 1-3, 6-8 or the ophthalmic composition for use according to claims 4-8, wherein said (b) verbascoside, isoverbascoside or mixtures is an extract of a plant of the species *Lippia citriodora,* preferably wherein said plant extract is a dry plant extract of the leaves of *Lippia citriodora.*

10. The ophthalmic composition according to claims 1-3, 6-9 or the ophthalmic composition for use according to claims 4-9, comprising (the following amounts being expressed as amount by weight of each component with respect to the overall weight of said composition, unless otherwise specified):

   (a.1) said hyaluronic acid or salt thereof, preferably sodium hyaluronate: from 0.05% to 5.0%, preferably from 0.1% to 2.0%, more preferably from 0.15% to 0.8%, even more preferably from 0.2% to 0.4%;
   (a.10) a polyethylene glycol (PEG) with an average molecular weight comprised from 200 Da to 600 Da, preferably PEG 300 or PEG 400, more preferably PEG 400: from 0.05% to 5.0%, preferably from 0.1% to 2.0%, more preferably from 0.15% to 0.8%, even more preferably from 0.2% to 0.4%;
   (b) verbascoside, isoverbascoside or mixtures thereof, preferably verbascoside, more preferably a dry plant extract of the leaves of *Lippia citriodora:* from 0.025% to 3.0%, preferably from 0.05% to 1.5%, more preferably from 0.08% to 0.4%, even more preferably from 0.1% to 0.3%;
   (c.1) a preservative, preferably boric acid: from 0.1% to 10.0%, preferably from 0.2% to 4.0%, more preferably from 0.3% to 2.0%, even more preferably from 0.7% to 0.9%;
   (c.2) at least one buffering agent, preferably sodium tetraborate and/or potassium hydroxide: up to a pH value comprised from 6.8 to 7.4;
   (c.3) at least one osmotic pressure regulator, preferably selected from the group comprising or, alternatively, consisting of: potassium chloride, calcium chloride, magnesium chloride, sodium chloride, and mixtures thereof: up to an osmolality comprised from 250 mOsm/kg to 300 mOsm/kg;
   (d) water, preferably purified water: up to 100%;

   preferably wherein said composition is substantially free of phosphate ions, i.e. free of phosphate ions, monohydrogen phosphate ions and/or dihydrogen phosphate ions.

11. The ophthalmic composition according to claims 1-3, 6-10 or the ophthalmic composition for use according to claims 4-10, wherein said composition is in the form of liquid eye drops, or gel, cream or ointment eye drops, preferably in the form of liquid eye drops.

**Patentansprüche**

1. Ophthalmologische Zusammensetzung, umfassend eine Mischung, die Folgendes umfasst oder alternativ besteht aus:

   (a) mindestens ein(em) Gleitmittel, wobei das (a) Gleitmittel eine Mischung aus
   (a.1) einer Hyaluronsäure oder einem Salz davon und
   (a.10) einem Polyethylenglykol (PEG) mit einem durchschnittlichen Molekulargewicht von 200 Da bis 600 Da;
   (b) Verbascosid, Isoverbascosid oder Mischungen davon; und gegebenenfalls
   (c) mindestens ein(em) pharmazeutisch akzeptablen Zusatzstoff.

2. Ophthalmologische Zusammensetzung nach Anspruch 1, wobei die (a.1) Hyaluronsäure oder ein Salz davon bevorzugt ein mittleres Molekulargewicht von 5.000 kDa bis 10.000 kDa, bevorzugt von 6.000 kDa bis 8.000 kDa, bevorzugter von 6.500 kDa bis 7.500 kDa, noch bevorzugter von 6.800 kDa bis 7.200 kDa aufweist.

3. Ophthalmologische Zusammensetzung nach Anspruch 1 oder 2, wobei die (a.1) Hyaluronsäure oder das Salz davon mit 1-Ethyl-3-(3-dimethylaminopropyl) carbodiimidhydrochlorid (EDC) vernetzt ist.

4. Ophthalmologische Zusammensetzung nach einem der Ansprüche 1-3, wobei die Zusammensetzung zur Verwendung in einem Verfahren zur präventiven oder kurativen Behandlung einer Erkrankung oder einer Krankheit, die mit einem Hornhautepitheldefekt und/oder einem Bindehautdefekt verknüpft ist, bei einem Patienten ist, die aus der Gruppe ausgewählt ist, die Folgendes umfasst oder alternativ daraus besteht: trockene(r) Keratitis, Xerophthalmie, Syndrom des trockenen Auges (Keratoconjunctivitis sicca), Sjögren-Syndrom und Stevens-Johnson-Syndrom.

5. Ophthalmologische Zusammensetzung zur Verwendung nach Anspruch 4, wobei die Erkrankung oder die Krankheit durch Luftverschmutzung, vorzugsweise durch städtische Staub- (UD-) Verschmutzung, verursacht wird.

6. Ophthalmologische Zusammensetzung nach einem der Ansprüche 1-3 oder ophthalmologische Zusammensetzung zur Verwendung nach den Ansprüchen 4 und 5, wobei ein Gewichtsverhältnis (a):(b) von 10:1 bis 1:5, bevorzugt von 5:1 bis 1:2, bevorzugter von 3:1 bis 1:1 liegt, noch bevorzugter 2:1 beträgt.

7. Ophthalmologische Zusammensetzung nach den Ansprüchen 1-3, 6 oder ophthalmologische Zusammensetzung zur Verwendung nach den Ansprüchen 4-6, wobei das (a) Gleitmittel eine Mischung aus (a.1) einer Hyaluronsäure oder einem Salz davon; und (a.10) einem Polyethylenglykol (PEG) mit einem durchschnittlichen Molekulargewicht von 200 Da bis 600 Da, bevorzugt von 250 Da bis 450 Da, bevorzugter von 280 Da bis 430 Da, noch bevorzugter von 300 Da (PEG 300) und/oder 400 Da (PEG 400) ist.

8. Ophthalmologische Zusammensetzung nach den Ansprüchen 1-3, 6, 7 oder ophthalmologische Zusammensetzung zur Verwendung nach den Ansprüchen 4-7, wobei ein Gewichtsverhältnis (a.1):(a.10) bevorzugt von 5:1 bis 1:5, bevorzugt von 2:1 bis 1:2, bevorzugter von 1,5:1 bis 1:1,5 liegt, noch bevorzugter 1:1 beträgt.

9. Ophthalmologische Zusammensetzung nach den Ansprüchen 1-3, 6-8 oder ophthalmologische Zusammensetzung zur Verwendung nach den Ansprüchen 4-8, wobei das (b) Verbascosid, Isoverbascosid oder Mischungen ein Extrakt einer Pflanze der Art *Lippia citriodora* ist, wobei der Pflanzenextrakt vorzugsweise ein trockener Pflanzenextrakt der Blätter von *Lippia citriodora* ist.

10. Ophthalmologische Zusammensetzung nach den Ansprüchen 1-3, 6-9 oder ophthalmologische Zusammensetzung zur Verwendung nach den Ansprüchen 4-9, umfassend (die folgenden Mengen werden als Gewichtsmenge einer jeden Komponente in Bezug auf das Gesamtgewicht der Zusammensetzung ausgedrückt, sofern nicht anders angegeben):

   (a.1) die Hyaluronsäure oder ein Salz davon, bevorzugt Natriumhyaluronat: von 0,05 % bis 5,0 %, bevorzugt von 0,1 % bis 2,0 %, bevorzugter von 0,15 % bis 0,8 %, noch bevorzugter von 0,2 % bis 0,4 %;
   (a.10) ein Polyethylenglykol (PEG) mit einem durchschnittlichen Molekulargewicht von 200 Da bis 600 Da, bevorzugt PEG 300 oder PEG 400, bevorzugter PEG 400: von 0,05 % bis 5,0 %, bevorzugt von 0,1 % bis 2,0 %, bevorzugter von 0,15 % bis 0,8 %, noch bevorzugter von 0,2 % bis 0,4 %;
   (b) Verbascosid, Isoverbascosid oder Mischungen davon, bevorzugt Verbascosid, bevorzugter ein trockener Pflanzenextrakt der Blätter von *Lippia citriodora:* von 0,025 % bis 3,0 %, bevorzugt von 0,05 % bis 1,5 %, bevorzugter von 0,08 % bis 0,4 %, noch bevorzugter von 0,1 % bis 0,3 %;
   (c.1) ein Konservierungsmittel, bevorzugt Borsäure: von 0,1 % bis 10,0 %, bevorzugt von 0,2 % bis 4,0 %, bevorzugter von 0,3 % bis 2,0 %, noch bevorzugter von 0,7 % bis 0,9 %;
   (c.2) mindestens ein Puffermittel, vorzugsweise Natriumtetraborat und/oder Kaliumhydroxid: bis zu einem pH-Wert von 6,8 bis 7,4;
   (c.3) mindestens einen osmotischen Druckregler, vorzugsweise ausgewählt aus der Gruppe umfassend oder alternativ bestehend aus: Kaliumchlorid, Calciumchlorid, Magnesiumchlorid, Natriumchlorid und Mischungen davon: bis zu einer Osmolalität von 250 mOsm/kg bis 300 mOsm/kg;
   (d) Wasser, vorzugsweise gereinigtes Wasser: bis zu 100 %;

   vorzugsweise wobei die Zusammensetzung im Wesentlichen frei von Phosphationen, d. h. frei von Phosphationen, Monohydrogenphosphationen und/oder Dihydrogenphosphationen ist.

**11.** Ophthalmologische Zusammensetzung nach den Ansprüchen 1-3, 6-10 oder ophthalmische Zusammensetzung zur Verwendung nach den Ansprüchen 4-10, wobei die Zusammensetzung in Form von flüssigen Augentropfen oder Gel-, Creme- oder Salben-Augentropfen, vorzugsweise in Form von flüssigen Augentropfen, vorliegt.

**Revendications**

**1.** Composition ophtalmique, comprenant un mélange qui comprend ou, en variante, est constituée :

> (a) d'au moins un agent lubrifiant, dans laquelle ledit agent lubrifiant (a) est un mélange
> (a.1) d'un acide hyaluronique ou un sel de celui-ci et
> (a.10) d'un polyéthylène glycol (PEG) ayant un poids moléculaire moyen compris de 200 Da à 600 Da ;
> (b) de verbascoside, de l'isoverbascoside ou des mélanges de ceux-ci ; et éventuellement
> (c) au moins un additif pharmaceutiquement acceptable.

**2.** Composition ophtalmique selon la revendication 1, dans laquelle ledit acide hyaluronique (a.1) ou un sel de celui-ci a de préférence un poids moléculaire moyen compris de 5 000 kDa à 10 000 kDa, de préférence compris de 6 000 kDa à 8 000 kDa, plus préférablement compris de 6 500 kDa à 7 500 kDa, encore plus préférablement compris de 6 800 kDa à 7 200 kDa.

**3.** Composition ophtalmique selon la revendication 1 ou 2, dans laquelle ledit acide hyaluronique (a.1) ou sel de celui-ci est réticulé avec du chlorhydrate de 1-éthyl-3-(3-diméthylaminopropyl)carbodiimide (EDC).

**4.** Composition ophtalmique selon l'une quelconque des revendications 1-3, dans laquelle ladite composition est destinée à être utilisée dans un procédé de traitement préventif ou curatif d'un trouble ou d'une maladie associé(e) à un défaut de l'épithélium cornéen et/ou à un défaut conjonctival, chez un sujet, choisi dans le groupe comprenant ou, en variante, constitué de : kératite sèche, xérophtalmie, syndrome de l'oeil sec (kératoconjonctivite sèche), syndrome de Sjögren et syndrome de Stevens-Johnson.

**5.** Composition ophtalmique destinée à être utilisée selon la revendication 4, dans laquelle ledit trouble ou ladite maladie est causé(e) par la pollution atmosphérique, de préférence par la pollution causée par les poussières urbaines (UD).

**6.** Composition ophtalmique selon l'une quelconque des revendications 1-3, ou composition ophtalmique destinée à être utilisée selon les revendications 4 et 5, dans laquelle le rapport pondéral (a):(b) est compris de 10:1 à 1:5, de préférence compris de 5:1 à 1:2, plus préférablement compris de 3:1 à 1:1, et encore plus préférablement est de 2:1.

**7.** Composition ophtalmique selon l'une quelconque des revendications 1-3, 6 ou composition ophtalmique destinée à être utilisée selon les revendications 4-6, dans laquelle ledit agent lubrifiant (a) est un mélange d'un acide hyaluronique (a.1) ou un sel de celui-ci ; et un polyéthylène glycol (PEG) (a.10) ayant un poids moléculaire moyen compris de 200 Da à 600 Da, de préférence compris de 250 Da à 450 Da, plus préférablement compris de 280 Da à 430 Da, encore plus préférablement de 300 Da (PEG 300) et/ou 400 Da (PEG 400).

**8.** Composition ophtalmique selon les revendications 1-3, 6, 7 ou composition ophtalmique destinée à être utilisée selon les revendications 4-7, dans laquelle un rapport pondéral (a.1) : (a.10) est de préférence compris de 5:1 à 1:5, de préférence compris de 2:1 à 1:2, plus préférablement compris de 1,5:1 à 1:1,5, et encore plus préférablement est de 1:1.

**9.** Composition ophtalmique selon les revendications 1-3, 6-8, ou composition ophtalmique destinée à être utilisée selon les revendications 4-8, dans laquelle ledit (b) verbascoside, isoverbascoside ou leurs mélanges est un extrait d'une plante de l'espèce *Lippia citriodora,* de préférence dans laquelle ledit extrait végétal est un extrait végétal sec des feuilles de *Lippia citriodora.*

**10.** Composition ophtalmique selon les revendications 1-3, 6-9 ou composition ophtalmique destinée à être utilisée selon les revendications 4-9, comprenant (les quantités suivantes étant exprimées en poids de chaque composant par rapport au poids total de ladite composition, sauf indication contraire) :

> (a.1) ledit acide hyaluronique ou sel de celui-ci, de préférence le hyaluronate de sodium : de 0,05 à 5,0 %, de préférence de 0,1 à 2,0 %, plus préférablement de 0,15 à 0,8 %, encore plus préférablement de 0,2 à 0,4 % ;

(a.10) un polyéthylène glycol (PEG) ayant un poids moléculaire moyen compris de 200 Da à 600 Da, de préférence PEG 300 ou PEG 400, plus préférablement PEG 400 : de 0,05 à 5,0 %, de préférence de 0,1 à 2,0 %, plus préférablement de 0,15 à 0,8 %, encore plus préférablement de 0,2 à 0,4 % ;

(b) du verbascoside, isoverbascoside ou leurs mélanges, de préférence le verbascoside, plus préférablement un extrait végétal sec des feuilles de *Lippia citriodora* : de 0,025 à 3,0 %, de préférence de 0,05 à 1,5 %, plus préférablement de 0,08 à 0,4 %, encore plus préférablement de 0,1 à 0,3 % ;

(c.1) un conservateur, de préférence de l'acide borique : de 0,1 à 10,0 %, de préférence de 0,2 à 4,0 %, plus préférablement de 0,3 à 2,0 %, encore plus préférablement de 0,7 à 0,9 % ;

(c.2) au moins un agent tampon, de préférence du tétraborate de sodium et/ou de l'hydroxyde de potassium : jusqu'à une valeur de pH comprise entre 6,8 et 7,4 ;

(c.3) au moins un régulateur de pression osmotique, de préférence choisi dans le groupe comprenant ou, en variante, constitué de : chlorure de potassium, chlorure de calcium, chlorure de magnésium, chlorure de sodium et leurs mélanges : jusqu'à une osmolalité comprise de 250 mOsm/kg à 300 mOsm/kg ;

(d) de l'eau, de préférence de l'eau purifiée : jusqu'à 100 % ;

de préférence, dans laquelle ladite composition est sensiblement exempte d'ions phosphate, c'est-à-dire exempte d'ions phosphate, d'ions monohydrogénophosphate et/ou d'ions dihydrogénophosphate.

11. Composition ophtalmique selon les revendications 1-3, 6-10 ou composition ophtalmique destinée à être utilisée selon les revendications 4-10, dans laquelle ladite composition se présente sous la forme de gouttes ophtalmiques liquides, ou de gouttes ophtalmiques sous forme de gel, de crème ou de pommade, de préférence sous la forme de gouttes ophtalmiques liquides.

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012158591 A1
- IT MI20091165 A1

**Non-patent literature cited in the description**

- *CHEMICAL ABSTRACTS*, 9004-64-03
- *CHEMICAL ABSTRACTS*, 74811-65-7
- *CHEMICAL ABSTRACTS*, 9004-32-4
- *CHEMICAL ABSTRACTS*, 9004-62-0
- *CHEMICAL ABSTRACTS*, 9004-67-5
- *CHEMICAL ABSTRACTS*, 9003-39-8
- *CHEMICAL ABSTRACTS*, 9003-04-7
- *CHEMICAL ABSTRACTS*, 8006-54-0
- *CHEMICAL ABSTRACTS*, 8012-95-1
- *CHEMICAL ABSTRACTS*, 8042-47-5
- *CHEMICAL ABSTRACTS*, 8002-74-2
- *CHEMICAL ABSTRACTS*, 8012-89-3
- *CHEMICAL ABSTRACTS*, 25322-68-3
- Handbook of Pharmaceutical Excipients
- *CHEMICAL ABSTRACTS*, 57-55-6
- *CHEMICAL ABSTRACTS*, 56-81-5
- *CHEMICAL ABSTRACTS*, 9005-65-6
- *CHEMICAL ABSTRACTS*, 1303-96-4
- *CHEMICAL ABSTRACTS*, 7447-40-7
- *CHEMICAL ABSTRACTS*, 10035-04-8
- *CHEMICAL ABSTRACTS*, 10043-52-4
- *CHEMICAL ABSTRACTS*, 7791-18-6
- *CHEMICAL ABSTRACTS*, 7647-14-5
- **RAMSAY E** ; **DEL AMO EM** ; **TOROPAINEN E** ; **TENGVALL-UNADIKE U** ; **RANTA VP** ; **URTTI A** ; **RUPONEN M**. Corneal and conjunctival drug permeability: Systematic comparison and pharmacokinetic impact in the eye. *Eur J Pharm Sci.*, July 2018